# EUROPEAN PATENT APPLICATION

(11) **EP 2 952 160 A1**
(43) Date of publication of application: **09.12.2015**
(21) Application number: 13873203.7
(22) Date of filing: 30.01.2013
(51) Int. Cl.: A61F 2/82

(54) **ORGANISM LUMEN TREATMENT SYSTEM, AND STENT**

(71) Applicant: Terumo Kabushiki Kaisha, Tokyo 151-0072 (JP)
(72) Inventor: MASUBUCHI, Yuki, Ashigarakami-gun Kanagawa 259-0151 (JP)
(74) Representative: Casalonga
(86) International application number: PCT/JP2013/051984
(87) International publication number: WO 2014/118905

(57) **Abstract**

A living body lumen treatment system (10) includes a sheath (16) that internally has a receiving lumen (24), and a stent (12) that is received in a contraction state by the receiving lumen (24) and that expands by being exposed from the sheath (16). A first region (70) to which a drug is applied and a second region (72) to which the drug is not applied are set to be located on an outer surface of the stent (12) . The sheath (16) has a protrusion portion (60) protruding beyond an inner wall (26). In the stent (12), the second region (72) is brought into contact with and supported by the protrusion portion (60) so as not to bring the first region (70) into contact with the inner wall (26).

## Description

### Technical Field

The present invention relates to a living body lumen treatment system which can treat a lesion inside a living body lumen by being delivered into the living body lumen, and additionally relates to a stent which is indwelled in the lesion by using the living body lumen treatment system.

### Background Art

Conventionally, stent treatment for causing a drug-applied stent (structure body) to be indwelled in a living body lumen by using a stent delivery device has been carried out for a lesion appearing inside the living body lumen such as blood vessels, biliary ducts, trachea, esophagi, urethrae and the like. For example, when intravascular stenosis is treated, the stent to which an immunosuppressive agent serving as a drug is applied is caused to be indwelled the intravascular stenosis, and the drug is eluted from the stent after the stent is indwelled, thereby preventing restenosis.

In addition to a balloon-expandable type whose expansion is operated by a balloon, a self-expandable type which can automatically expand is known as the stent indwelled in the stenosis. The self-expandable stent has a predetermined elastic force. When being delivered into the blood vessel, the stent contracts by being pressed inward by a sheath (tubular body) of the device, and expands by being exposed from the sheath (for example, refer to JP-A-2012-55484).

Incidentally, when the drug-applied self-expandable stent is used, in a state where the sheath is received, the drug applied to an outer peripheral surface of the stent comes into contact with an inner wall of the sheath. Therefore, when being received by the sheath, while being received in the sheath, or when being exposed from the sheath, the self-expandable stent comes into physical contact (compression, scraping and the like) with the sheath, thereby causing a disadvantage in that the drug is peeled off (detached. Since the drug is peeled off from the stent in this way, the drug cannot be sufficiently eluted after the stent is indwelled, thereby causing a problem in that the treatment is adversely affected.

### Summary of Invention

The present invention is made in view of the above-described circumstances, and an object thereof is to provide a living body lumen treatment system and a stent which can considerably prevent a drug from being detached when a drug-applied structure body is delivered by being received inside a tubular body and is deployed inside a living body lumen, and which accordingly enables satisfactory treatment for the living body lumen.

In order to achieve the above-described object, according to the present invention, there is provided a living body lumen treatment system including a tubular body that can be delivered into a living body lumen, and that internally has a lumen extending in an axial direction, and a structure body that is received in a contraction state by the lumen, and that expands by being exposed from the tubular body so as to come into contact with an inner surface of the living body lumen. A first region to which a drug is applied and a second region to which an application agent having an application form different from the drug is applied or to which the drug is not applied are set to be located on an outer surface of the structure body. The tubular body has a protrusion portion protruding inward beyond an inner wall which configures the lumen. The structure body is received by the lumen in such a way that the second region is brought into contact with and supported by the protrusion portion so as not to bring the first region into contact with the inner wall.

According to the above-described configuration, the first region to which the drug is applied and the second region to which the application agent having the application form different from the drug is applied or to which the drug is not applied are set to be located on the structure body. Only the second region is brought into contact with and supported by the protrusion portion of the tubular body (in other words, the first region is not brought into contact with the inner wall of the tubular body). In this manner, the living body lumen treatment system can considerably minimize the detachment of the drug. That is, the first region of the structure body is not brought into contact with the inner wall of the tubular body. Accordingly, the living body lumen treatment system can deploy the structure body inside the living body lumen while suppressing the detachment of the drug which may be caused by the structure being in contact with the inner wall. Therefore, the drug can be stably eluted or attached into the living body lumen from the structure body while maintaining the quality of the drug. Accordingly, it is possible to satisfactorily treat the living body lumen.

In this case, preferably, the protrusion portion has a total length longer than a length in an axial direction of the structure body, and is disposed along the axial direction of the tubular body. Preferably, the first region and the second region extend along the axial direction of the structure body, and are set at positions deviated from each other in a circumferential direction of the structure body.

In this manner, the first region and the second region extend along the axial direction of the structure body, and are set to be located at the positions deviated from each other in the circumferential direction of the structure body. Accordingly, when the structure body and the tubular body are relatively moved forward and rearward in the axial direction, it is possible to continuously maintain a state where the second region is brought into contact with and supported by the protrusion portion, and a state where the first region is not brought into contact with the inner wall. Therefore, it is possible to more reliably prevent the drug from being detached.

In addition, preferably, a surface of the protrusion portion that comes into contact with the second region has a friction force lower than the inner wall.

In this manner, since the friction force of the protrusion portion is lower than the friction force of the inner wall, the tubular body can be smoothly moved forward and rearward relative to the structure body. Therefore, it is possible to efficiently deploy the structure body.

Here, when the application agent is applied to the second region, the application agent may satisfy at least one of physical properties selected from a lower friction force, higher elasticity, or higher rigidity, as compared to the drug.

In this manner, since the application agent satisfies the physical properties selected from the lower friction force, the higher elasticity, or the higher rigidity, the protrusion portion can be easily deformed with respect to the second region to which the application agent is applied. Therefore, the structure body can be more smoothly moved forward and rearward.

Furthermore, the structure body may be a stent which is configured to include an elastically deformable wire, and accordingly the stent is pressed and brought into a contraction state by the tubular body when the stent is received inside the tubular body. The second region may be set to be located at a place where a distortion amount of the wire is large in the contraction state.

That is, the place where the distortion amount of the wire is large in the contraction state of the structure body corresponds to a place receiving a strong pressing force from the inner wall of the tubular body. Accordingly, since the second region is set to be located in the place where the distortion amount is large, the structure body can be smoothly deployed into the living body lumen. Therefore, it is possible to more reliably prevent the drug from being detached.

In addition, in order to achieve the above-described object, according to the present invention, there is provided a stent that is received in a contraction state by a lumen of a tubular body which can be delivered into a living body lumen, and that expands by being exposed from the tubular body so as to be indwelled in the living body lumen. A first region to which a drug is applied and a second region to which an application agent having an application form different from the drug is applied or to which the drug is not applied are set to be located on an outer surface of the stent. The stent is received by the lumen in such a way that the second region is brought into contact with and supported by a protrusion portion protruding inward beyond an inner wall configuring the lumen so as not to bring the first region into contact with the inner wall.

In this case, the first region and the second region may extend along an axial direction of the stent, and may be set at positions deviated from each other in a circumferential direction of the stent.

In addition, when the application agent is applied to the second region, preferably, the application agent satisfies at least one of physical properties selected from a lower friction force, higher elasticity, or higher rigidity, as compared to the drug.

Furthermore, the stent may be configured to include an elastically deformable wire, and accordingly the stent may be pressed and brought into a contraction state by the tubular body when the stent is received inside the tubular body. The second regionmaybe set to be located at a place where a distortion amount of the wire is large in the contraction state.

### Brief Description of Drawings

[Fig. 1] Fig. 1 is a perspective view illustrating an overall configuration of a living body lumen treatment system according to an embodiment.
[Fig. 2] Fig. 2 is a side cross-sectional view illustrating a distal side of a sheath in Fig. 1.
[Fig. 3] Fig. 3A is a deployment view where a stent in a contraction state in Fig. 1 is deployed in a circumferential direction, and Fig. 3B is a deployment view where the stent in an expansion state in Fig. 3A is deployed in the circumferential direction.
[Fig. 4] Fig. 4A is an explanatory view for illustrating a relationship between the stent in Fig. 3A and a protrusion portion, and Fig. 4B is an explanatory view for illustrating an enlarged portion of the stent in Fig. 4A.
[Fig. 5] Fig. 5A is a cross-sectional view taken along line VA-VA in Fig. 4A, Fig. 5B is a cross-sectional deployment view taken along line VB-VB in Fig. 4A, and Fig. 5C is a cross-sectional deployment view taken along line VC-VC in Fig. 4A.
[Fig. 6] Fig. 6A is a cross-sectional deployment view illustrating a stent according to a first configuration example, Fig. 6B is a cross-sectional deployment view illustrating a stent according to a second configuration example, and Fig. 6C is a cross-sectional deployment view illustrating a stent according to a third configuration example.
[Fig. 7] Fig. 7A is an explanatory view for illustrating a relationship between a stent and a protrusion portion of a living body lumen treatment system according to a first modification example, and Fig. 7B is an explanatory view for illustrating a relationship between a stent and a protrusion portion of a living body lumen treatment system according to a second modification example.

### Description of Embodiments

Hereinafter, a living body lumen treatment system and a stent according to the present invention will be described in detail with reference to preferred embodiments and the accompanying drawings.

The living body lumen treatment system is used when an interventional technique inside a living body lumen is applied to a lesion appearing inside the living body lumen such as blood vessels, biliary ducts, trachea, esophagi, urethrae and the like. In particular, in the present embodiment, the living body lumen treatment system which causes the stent to be indwelled in stenosis (lesion) appearing in the blood vessel which is the living body lumen will be described in detail.

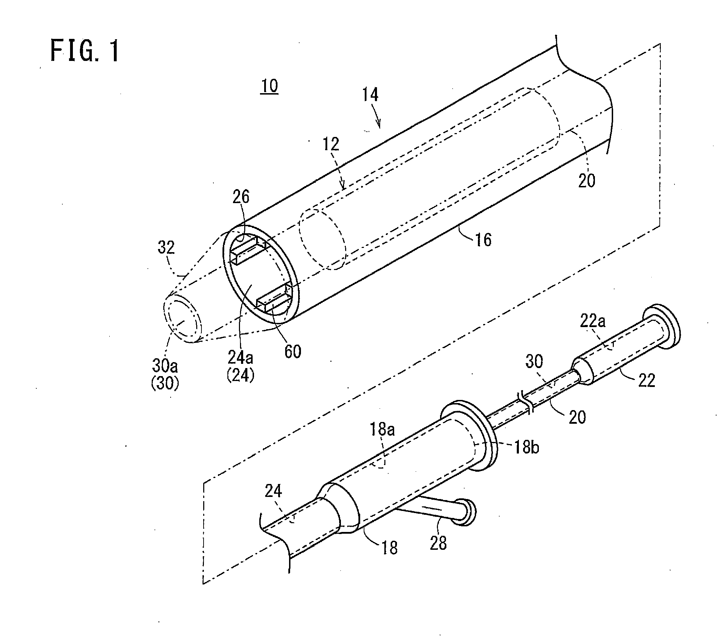

As illustrated in Fig. 1, a living body lumen treatment system 10 (hereinafter, simply referred to as a system 10) is configured to include a stent 12 (indwelled object: structure body) indwelled in the stenosis, and a stent delivery device 14 (catheter) for delivering and indwelling of the stent 12.

The device 14 includes a sheath 16 having enough length to be delivered into the stenosis inside the blood vessel, a sheath hub 18 connected to a proximal portion of the sheath 16, a shaft 20 which is inserted into the sheath 16 and in which the stent 12 is placed at a predetermined position, and a shaft hub 22 connected to a proximal portion of the shaft 20.

The sheath 16 is an flexible tubular body which can be inserted and delivered into the blood vessel, and has a receiving lumen 24 (lumen) formed to penetrate in an axial direction (longitudinal direction). The stent 12 is received by the receiving lumen 24 on the distal side of the sheath 16, and thus is guided to the stenosis inside the blood vessel by the device 14. The receiving lumen 24 is configured so that the surrounding area thereof is surrounded by an inner wall 26 of the sheath 16, and extends into the sheath 16 while maintaining a substantially constant inner diameter. A distal opening 24a formed on a distal surface of the sheath 16 communicates with the receiving lumen 24, in which the shaft 20 is arranged so as to be movable forward and rearward.

Preferably, the sheath 16 has suitable flexibility and suitable strength (rigidity) so that the distal portion thereof can be smoothly delivered into the stenosis inside the blood vessel. For example, as a material for configuring the sheath 16, it is possible to preferably employ, polyolefin such as polyethylene, polypropylene and the like, polyamide (nylon), polyester such as polyethylene terephthalate and the like, fluorinated polymers such as PTFE, ETFE and the like, PEEK (polyether ether ketone), polyimide, and thermoplastic elastomer such as polyamide elastomer, polyester elastomer and the like. Note that an outer surface on the distal side of the sheath 16 may be coated with resin that has biocompatibility, particularly, antithrombogenicity. For example, as an antithrombotic material, it is preferable to employ poly-hydroxyethyl methacrylate, a copolymer of hydroxyethyl methacrylate and styrene (for example, HEMA-St-HEMA block copolymer), and the like. In addition, when the device 14 is used, in order to confirm a position of the sheath 16 inside the blood vessel under X-ray fluoroscopy, a contrast marker (not illustrated) may be disposed at a distal end of the sheath 16.

The sheath hub 18 connected to the proximal portion of the sheath 16 is formed to have a larger diameter than the sheath 16, and functions as an operation unit for delivering the distal portion of the sheath 16 inside the blood vessel. The sheath hub 18 internally has a cavity portion 18a for communicating with the receiving lumen 24, and a port 28 having a passage (not illustrated) for communicating with the cavity portion 18a is formed on an outer peripheral surface thereof.

The port 28 is used to supply a priming solution (for example, a physiological salt solution) to the receiving lumen 24 during priming or to supply a contrast medium after being inserted into the blood vessel. The cavity portion 18a communicates with a proximal opening 18b of the sheath hub 18, and the shaft 20 to be received by the receiving lumen 24 is inserted into the proximal opening 18b.

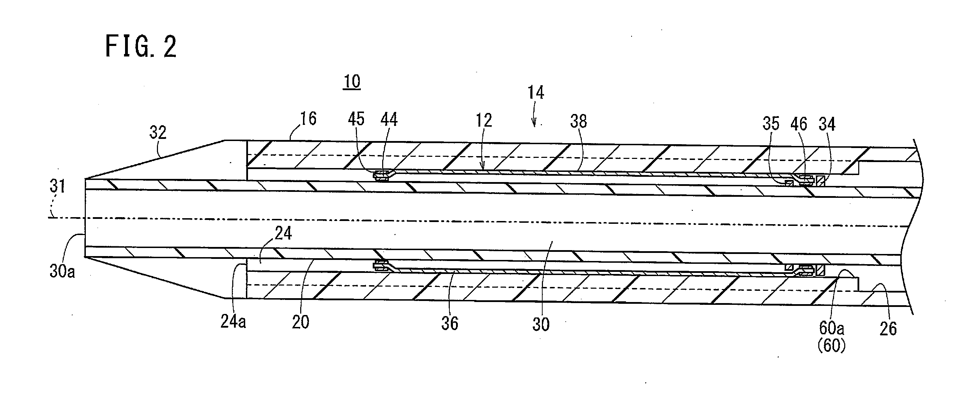

The shaft 20 is formed to be longer than the axial length (total length) of the sheath 16. The shaft 20 is exposed from the distal portion of the sheath 16 in a state where the shaft 20 is received by the receiving lumen 24, and is exposed from the proximal opening 18b of the sheath hub 18. A guidewire lumen 30 is formed to penetrate in the axial direction inside the shaft 20. A guidewire 31 (refer to Fig. 2) is inserted into the guidewire lumen 30.

The shaft hub 22 connected to the proximal portion of the shaft 20 has a function to operate the shaft 20 separately from the sheath 16. A cavity portion 22a for communicating with the guidewire lumen 30 is formed inside the shaft hub 22. That is, the device 14 is a catheter of an over-the-wire type into which the guidewire 31 is inserted along the axial direction of the shaft 20 from the shaft hub 22 (cavity portion 22a). As a matter of course, without being limited to this configuration, the device 14 can employ a rapid exchange type in which the guidewire 31 is exposed outward from the sheath 16 at an intermediate position of the sheath 16.

In addition, a nose cone 32 configuring the distal portion of the device 14 is disposed on an outer peripheral surface of the distal portion of the shaft 20. The shaft 20 penetrates a central portion of the nose cone 32, and a wire feeding port 30a for communicating with the guidewire lumen 30 is formed on a distal surface of the nose cone 32. A proximal surface of the nose cone 32 is formed in a flat shape and can come into liquid-tight contact with the distal surface of the sheath 16. Therefore, when being delivered inside the blood vessel, the distal opening 24a of the receiving lumen 24 is closed, thereby preventing the blood from flowing backward into the receiving lumen 24, for example.

In addition, as illustrated in Fig. 2, a pair of stent locking portions 34 and 35 for regulating a movement in the axial direction of the stent 12 are disposed on the outer peripheral surface of the shaft 20 on which the stent 12 is placed. The pair of stent locking portions 34 and 35 are separated from each other at an interval which is the same as the axial length of a proximal side decreased diameter portion 46 of the stent 12. Front and rear portions of the proximal side decreased diameter portion 46 are configured to be interposed between the pair of stent locking portions 34 and 35. The pair of stent locking portions 34 and 35 come into contact with the proximal side decreased diameter portion 46 when the sheath 16 and the shaft 20 are moved relatively, thereby preventing the stent 12 from being misaligned in the axial direction.

The stent 12 of the system 10 has a self-expandable function, and is received in a space (receiving lumen 24) formed between the shaft 20 and the sheath 16, in a state where the stent is folded by the expansion being regulated (contraction state, expandable state). The stent 12 automatically expands to restore a previous shape before contraction, when the sheath 16 is moved rearward relative to the shaft 20 and the sheath 16 releases the expansion regulation.

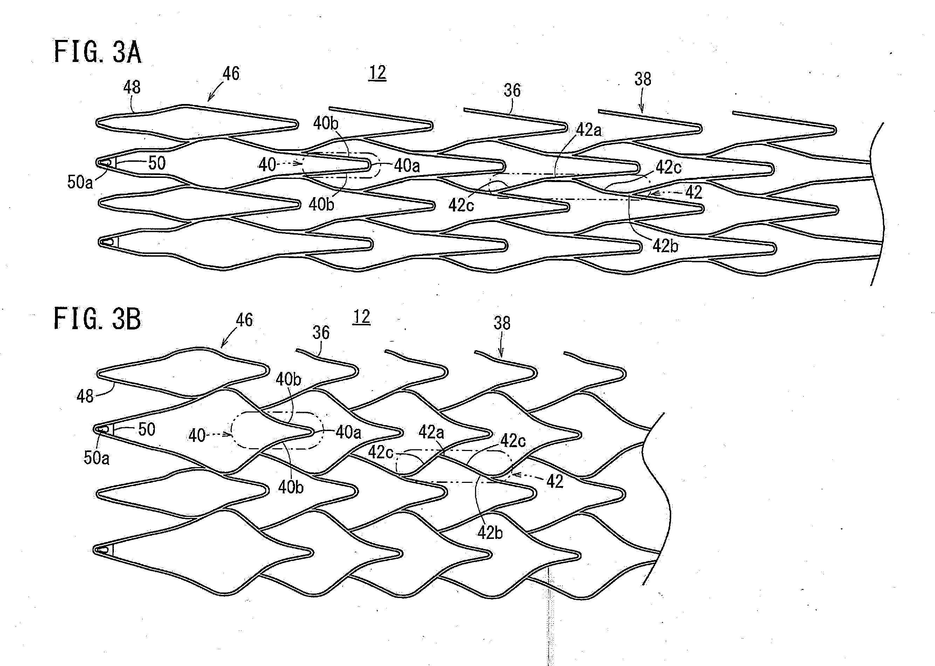

As illustrated in Figs. 3A and 3B, the stent 12 is configured to include a shape memory wire 36 (strut) which can restore a predetermined shape. The stent 12 includes a main tubular body portion 38 which has an outer diameter larger than an inner diameter of the indwelling-targeted blood vessel and has the axial length longer than a disease onset range of the lesion, in a natural state.

Themain tubular body portion 38 contracts radially inward and is elastically deformed so as to extend in the axial direction, in a contraction state. The main tubular body portion 38 has a V-shaped framework portion 40 and a wavy framework portion 42 which are formed in different shapes since the wire 36 is curved. The V-shaped framework portion 40 and the wavy framework portion 42 are alternately arranged along the circumferential direction. In addition, the multiple V-shaped framework portions 40 and the multiple wavy framework portions 42 are disposed to be respectively arrayed side by side along the axial direction. That is, the main tubular body portion 38 is formed in a tubular shape in such a way that a row of the V-shaped framework portion 40 and a row of the wavy framework portion 42 are alternately disposed in the circumferential direction.

One V-shaped framework portion 40 has a V-shaped lateral apex 40a and a pair of V-shaped lateral extension portions 40b and 40b connected to the V-shaped lateral apex 40a. One wavy framework portion 42 has wavy lateral apexes 42a and 42b undulating along the circumferential direction of the main tubular body portion 38, and a wavy lateral extension portion 42c curvedly connecting the wavy lateral apexes 42a and 42b to each other. The pair of V-shaped lateral extension portions 40b and 40b interconnect the V-shaped lateral apex 40a and the wavy lateral apexes 42a and 42b. This causes the row of the V-shaped framework portion 40 and the row of the wavy framework portion 42 to be connected to each other.

In a contraction state (refer to Fig. 3A), the stent 12 is elastically deformed so that the pair of V-shaped lateral extension portions 40b and 40b move close to each other. Accordingly, relatively greater stress is applied to the V-shaped lateral apex 40a (distortion amount is large). Therefore, when the stent 12 expands, the pair of V-shaped lateral extension portions 40b and 40b are elastically restored so as to be separated from each other (refer to Fig. 3B).

In addition, in the contraction state (refer to Fig. 3A), the wavy framework portion 42 of the stent 12 acts so as to extend in the axial direction, thereby increasing the radius of curvature of the wave further than that in a natural state. Specifically, the wavy lateral apexes 42a and 42b move close to each other, thereby bringing the wavy lateral extension portions 42c into a state of extending in the axial direction. Therefore, when the stent 12 expands, the wavy lateral apexes 42a and 42b are separated from each other, thereby causing the wavy lateral extension portions 42c to be elastically restored so as to move close to each other in the axial direction (refer to Fig. 3B).

Therefore, when a contraction state is switched over to an expansion state (automatically expanding), the main tubular body portion 38 of the stent 12 is allowed to smoothly expand outward in the radial direction and to smoothly contract in the axial direction. In addition, since the framework portions (V-shaped framework portion 40 and wavy framework portion 42) having the same shape are arrayed side by side along the axial direction, the stent 12 is likely to move forward to and rearward relative to the sheath 16.

A distal side decreased diameter portion 44 and a proximal side decreased diameter portion 46 which are formed to have a slightly smaller diameter than that of the main tubular body portion 38 are disposed in both ends (distal end and proximal end) in the axial direction of the main tubular body portion 38 (refer to Fig. 2). Note that, in the following description, the proximal side decreased diameter portion 4 6 will be described as a representative example with reference to Figs. 3A and 3B. Unless otherwise specifically indicated, description of the distal side decreased diameter portion 44 formed symmetrically will be omitted.

The proximal side decreased diameter portion 46 is formed in a tubular shape, is connected to the main tubular body portion 38, and has an extension portion 48 configuring a curved apex extending to the proximal side at a position opposing the V-shaped framework portion 40. The multiple extension portions 48 are disposed along the circumferential direction of the stent 12. A locking portion 50 having a locking hole 50a into which a rivet-shaped contrast marker can be inserted is formed in an apex of a predetermined extension portion 48 (every other apex). That is, in a contraction state, the stent 12 is placed on the outer peripheral surface of the shaft 20 by the rivet-shaped contrast marker being inserted into a portion between the pair of the stent locking portions 34 and 35.

It is preferable to configure the stent 12 by using a suitable material so that the stent 12 has a self-expandable elastic force (particularly super-elasticity) with the exposure from the sheath 16. For example, materials for configuring the stent 12 include various metals such as stainless steel, Ni-Ti alloy, Cu-Zn alloy, Ni-Al alloy, Co-Cr alloy, tungsten, tungsten alloy, titanium, titanium alloy, tantalum and the like; polymer materials having relatively high rigidity such as polyamide, polyimide, ultra-high molecular weight polyethylene, polypropylene, fluorine resin and the like; or a suitable combination of these materials; and the like. In addition, the stent 12 itself is configured to have an X-ray contrast property. Accordingly, after treatment, it is possible to confirm an indwelled state of the stent 12 inside the blood vessel.

Then, a drug M (immunosuppressive agent) for suppressing restenosis of the stenosis is applied to (coats) the wire 36 configuring the stent 12. For example, the immunosuppressive agent includes sirolimus, or sirolimus derivatives such as everolimus, pimecrolimus, ABT-578, AP23573, CCI-779 and the like, or tacrolimus, azathioprine, cyclosporine, cyclophosphamide, mycophenolate mofetil, gusperimus, mizoribine, and doxorubicin. Note that the drug M left in a state of being mixed with a biodegradable polymer such as polylactic acid and the like may be applied to the stent 12.

In addition, the drug M applied to the stent 12 is not limited to the immunosuppressive agent, and as amatter of course, various drugs may be applied to the stent 12 depending on treatment content. For example, other drugs include anti-cancer agents, antibiotics, anti-rheumatic agents, antithrombotic agents, HMG-CoA reductase inhibitors, insulin resistance-improving agents, ACE inhibitors, calcium antagonists, anti-hyperlipidemicagents, integrin inhibitors, anti-allergic agents, anti-oxidants, GPIIb/IIIa antagonists, retinoids, flavonoids, carotenoids, lipid improvers, DNA synthesis inhibitors, tyrosine kinase inhibitors, antiplatelet agents, anti-inflammatory agents, living body-derived materials, interferon, nitric oxide production-promoting substances and the like.

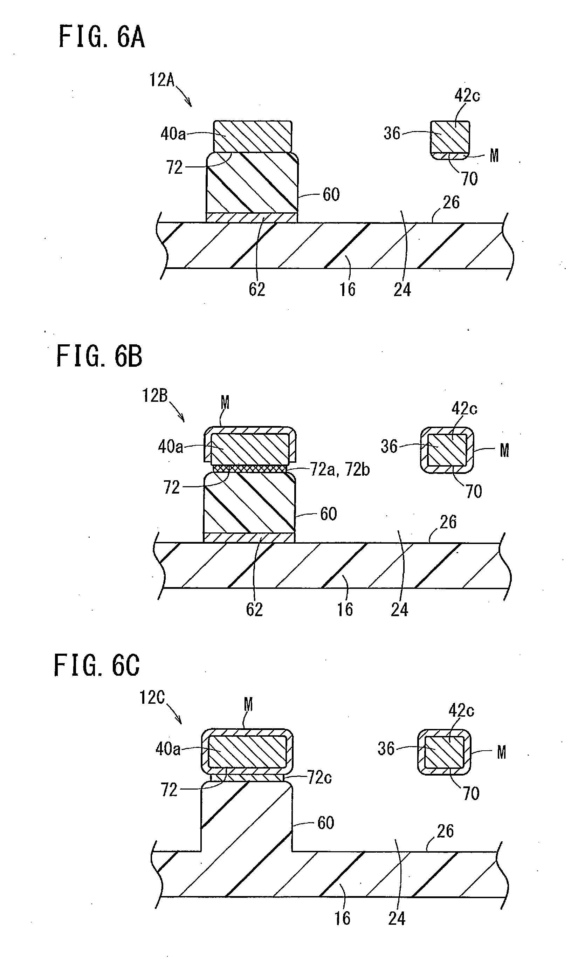

In a state where the stent 12 is indwelled in the blood vessel, the above-described drug M (immunosuppressive agent) is gradually eluted with the lapse of time. This can prevent vascular smooth muscle cells from growing, and can effectively prevent the restenosis of the blood vessel. It is preferable to apply the drug M to the entire surface (entire outer surface) of the wire 36 in a first region 70 (to be described later). However, for example, the drug M may be applied to only the outer peripheral surface side of the stent 12 configured to include the wire 36 (first configuration example, refer to Fig. 6A).

Then, the system 10 according to the present embodiment is configured so as to prevent the detachment of the drug M applied to the stent 12. Hereinafter, this configuration will be described specifically.

As illustrated in Figs. 1, 4A, 4B, and 5A to 5C, multiple (four in the present embodiment) protrusion portions 60 protruding to the axis of the sheath 16 are disposed on the inner wall 26 configuring the receiving lumen 24 of the sheath 16. The first region 70 (drug application region) to which the drug M is applied and a second region 72 (drug non-application region) to which the drug M is not applied are disposed on the outer peripheral surface of the stent 12.

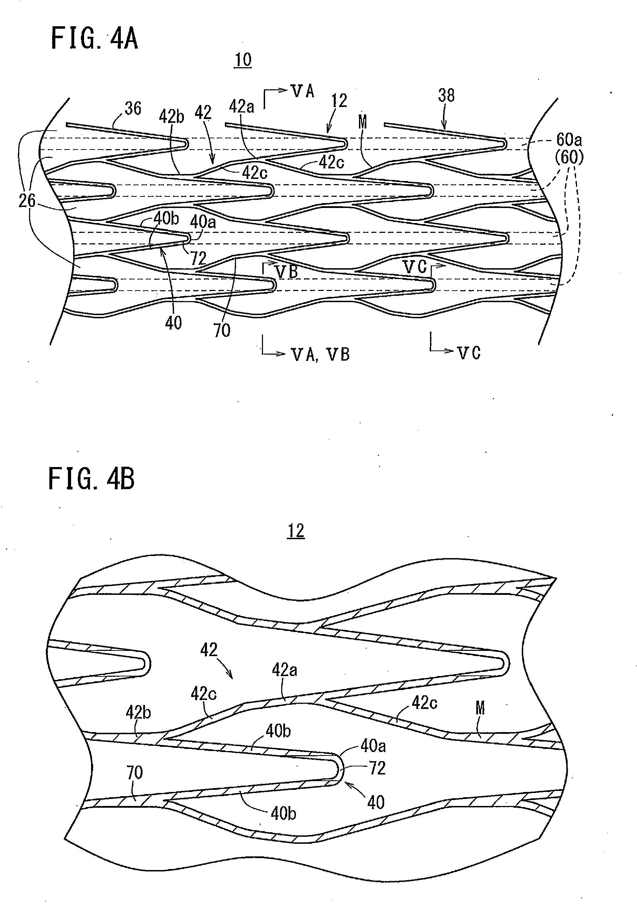

Four protrusion portions 60 are disposed at equal intervals (intervals of every 90 degrees) along the circumferential direction of the inner wall 26 of the sheath 16. In a deployment view (refer to Fig. 4A) in the circumferential direction of the sheath 16, the four protrusion portions 60 extend linearly and in parallel with each other along the axial direction of the sheath 16. In addition, each of the protrusion portions 60 is continuous to the distal portion of the sheath 16, is directed in the proximal end direction, and is formed over a range exceeding the receiving position of the stent 12 (also refer to Fig. 2). Therefore, the four protrusion portions 60 can support the stent 12 received by the receiving lumen 24 at intervals of 90 degrees. In addition, the four protrusion portions 60 function as a rib for reinforcing the distal portion of the sheath 16. Note that the protrusion portions 60 may be formed to extend to the proximal portion of the sheath 16.

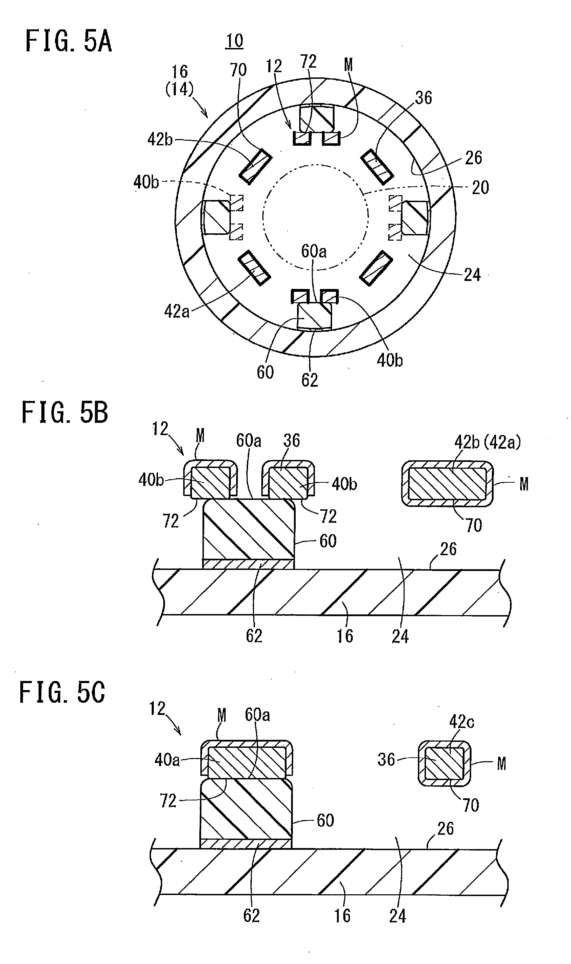

As illustrated in Figs. 5A to 5C, each of the protrusion portions 60 has a slightly wider width than the width of the wire 36 of the stent 12, and protrudes beyond the inner wall 26 toward the axis. The protrusion portions 60 are firmly and fixedly attached to the inner wall 26 via a binder layer 62. Materials for configuring the binder layer 62 are not particularly limited, and for example, may include synthetic adhesives such as acrylic resin-based adhesives, α-olefin-based adhesives, silicone-based adhesives, polyimide-based adhesives and the like, or organic type adhesives and the like. Note that a structure for installing the protrusion portion 60 in the sheath 16 is not limited to the bonding structure using the binder layer 62, and for example, the protrusion portion 60 may be integrally molded with the inner wall 26 of the sheath 16 (third configuration example: refer to Fig. 6C).

An upper surface of the protrusion portion 60 serves as a support surface 60a for supporting the stent 12 by directly coming into contact with the stent 12. The stent 12 vertically and laterally receives a pressing force from the support surface 60a, thereby while maintaining a contraction state, the stent 12 is received so that the sheath 16 (protrusion portion 60) freely moves forward and rearward relative to the stent 12. An edge portion of the support surface 60a is formed in a R-shape so as not to be caught thereon when the sheath 16 moves forward and rearward relative to the stent 12.

The support surface 60a of the protrusion portion 60 is configured to include a material whose friction force (friction coefficient) is lower than the inner wall 26 of the sheath 16 so that the sheath 16 can smoothly move forward and rearward relative to the stent 12. Materials for configuring the protrusion portion 60 depend on the material of the sheath 16, but for example, may include resin materials such as polyacetal (POM), polyamide (PA), polytetrafluoroethylene (PTFE), polyolefin, thermoplastic elastomer, thermosetting resin, other super engineering plastic and the like; metal; ceramics; and the like.

As a matter of course, configurations in which the periphery (outer surface including the support surface 60a) of the protrusion portion 60 is coated with a low friction material or a lubricant may be used, or the protrusion portion 60 to which the low friction material or the lubricant is blended may be used. In addition, the friction force may be decreased by smoothly processing the support surface 60a of the protrusion portion 60.

On the other hand, the stent 12 (main tubular body portion 38) is configured so that the outer peripheral surface is entirely covered with the multiple (four) first regions 70 and (four) second regions 72. The first regions 70 and the second regions 72 are appropriately set to be located depending on a relationship with the protrusion portion 60 arranged in the sheath 16.

Specifically, the four first regions 70 and the four second regions 72 are alternately arrayed side by side in the circumferential direction of the stent 12, and are set to extend linearly and in parallel with each other along the axial direction of the stent 12. That is, in a deployment view (refer to Figs. 4A and 4B) in the circumferential direction of the stent 12, the first regions 70 and the second regions 72 have a stripe shape.

The first regions 70 are set to be located in a wider range in the circumferential direction than the second regions 72. The second regions 72 are disposed at intervals of every 90 degrees in the circumferential direction so as to interpose the first regions 70 therebetween, and are set to be located so that the width in the circumferential direction is substantially coincident with the width of the protrusion portion 60. That is, the stent 12 is received inside the sheath 16 (receiving lumen 24) so that the first region 70 opposes the inner wall 26 and the second region 72 opposes the protrusion portion 60 (refer to Fig. 4A). According to this configuration, the second region 72 to which the drug M is not applied is supported by the protrusion portion 60.

Note that, as illustrated in Figs. 5A to 5C, in the stent 12, it is preferable to apply the drug M to an inner side portion of the second region 72. According to this configuration, it is possible to elute the drug M through the inner side portion after the stent 12 is indwelled. As a matter of course, the drug M may not be applied to the inner side portion of the second region 72 (first configuration example: refer to Fig. 6A).

Then, as illustrated in Fig. 4B, the second region 72 is set to be located in a range including the V-shaped lateral apex 40a of the V-shaped framework portion 40 and a part of the V-shaped lateral extension portion 40b (part close to the V-shaped lateral apex 40a). As described above, in a contraction state of the stent 12, the pair of V-shaped lateral extension portions 40b and 40b move close to each other, thereby causing the strongest stress to be applied to the V-shaped lateral apex 40a within the elastically deformed wire 36. That is, the V-shaped lateral apex 40a has a large distortion amount of the wire 36, and comes into contact with the sheath 16 with a relatively strong force (by receiving the pressing force).

When the stent 12 is received by the sheath 16, the V-shaped lateral apex 40a is supported by the support surface 60a of the protrusion portion 60. In this manner, other portions (other portions of the V-shaped lateral extension portion 40b and the wavy framework portion 42) can be reliably separated from the inner wall 26, and can be brought into a floating state inside the receiving lumen 24. Then, the second region 72 is set to be located in the V-shaped lateral apex 40a. In this manner, even when the second region 72 relatively strongly comes into contact with the protrusion portion 60, it is possible to prevent the drug M from being detached due to the protrusion portion 60.

On the other hand, the first region 70 is set to be located in other portions of the V-shaped lateral extension portion 40b and the wavy framework portion 42. The second region 72 is supported by the protrusion portion 60, and thus is not brought into contact with the inner wall 26. Therefore, it is possible to avoid a case where the sheath 16 interferes with the applied portion of the drug M.

The first region 70 and the second region 72 are arranged along an extension direction of the protrusion portion 60. Accordingly, even when the sheath 16 moves forward and rearward relative to the stent 12, the second region 72 always moves on the protrusion portion 60. Therefore, the stent 12 can be easily exposed from the sheath 16.

In addition, when the stent 12 is deployed (expands), the first region 70 includes other portions of the V-shaped lateral extension portion 40b and the wavy framework portion 42 which expand in the radial direction. In the stent 12 in an expansion state, the first region 70 configures a majority in the circumferential direction of the stent 12. That is, even when the second region 72 is set to be located in the V-shaped lateral apex 40a (and the close position), the second region 72 is limitedly located in a small range in the circumferential direction in the expansion state. Therefore, in a state where the stent 12 is indwelled, it is also possible to cover the second region 72 by eluting the drug M from the first region 70.

Note that, as in a stent 12A according to a first configuration example illustrated in Fig. 6A, a structure may be adopted in which the drug M is applied to only the outer peripheral surface side of the wire 36 in the first region 70 without applying the drug M to the wire 36 in the second region 72 at all.

Additionally, in addition to the configuration where the drug is not applied to the second region 72, an application agent which has an application form different from that of the drug M in the first region 70 may be applied to the second region 72. For example, a stent 12B according to a second configuration example illustrated in Fig. 6B is configured so that a lubricant 72a is applied to (coats) the second region 72. The lubricant 72a can decrease the friction force in the second region 72, thereby allowing the sheath 16 (protrusion portion 60) to more easily move forward and rearward relative to the stent 12. For example, the lubricant 72a includes hydrophilic polymer such as poly (2-hydroxyethyl methacrylate), poly hydroxyethyl acrylate, hydroxypropyl cellulose, methyl vinyl ether-maleic anhydride copolymer, polyethylene glycol, polyacrylamide, polyvinyl pyrrolidone, dimethylacrylamide-glycidyl methacrylate copolymer and the like.

In addition, the application agent applied to the second region 72 is not limited to the lubricant 72a. An application agent 72b which satisfies at least one of physical properties selected from a lower friction force, higher elasticity, and higher rigidity as compared to the drug M may be applied to the first region 70. The application agent 72b having these physical properties improves durability of the application agent 72b itself, and can decrease the adhesion between the protrusion portion 60 and the second region 72. Accordingly, it is possible to improve the mobility of the sheath 16 relative to the stent 12.

Alternatively, as the application agent, a drug (not illustrated) which satisfies any one of the physical properties such as the lower friction force, the higher elasticity, and the higher rigidity may be applied thereto by adopting a configuration using a composition different from that of the drug M of the first region 70. For example, a drug having a composition with higher rigidity (harder) than the drug M in the first region 70 is applied to the second region 72. In this manner, even when the second region 72 is brought into contact with and supported by the protrusion portion 60, it is possible to minimize the detachment of the drug.

Furthermore, as in a stent 12C according to a third configuration example illustrated in Fig. 6C, the drug M which is the same as that of the first region 70 may be applied to the second region 72 so as to have a multiple layer (two layer) structure in which a thin film application agent 72c (lubricant or the like) is applied to an upper layer thereof. For example, the application agent 72c is configured to include a polymer which is harder than the drug M. As an application form of the second region 72, the multiple layer structure is employed in this way. Accordingly, when the sheath 16 moves forward and rearward relative to the stent 12, or after the stent 12 is caused to be indwelled in a lesion, the application agent 72c applied to the upper layer is peeled off, thereby enabling the drug applied to the lower layer to be eluted.

The system 10 according to the present embodiment is basically configured as described above. Hereinafter, an operation and effect will be described.

The stent 12 is allowed to freely move forward and rearward in the axial direction integrally with the shaft 20 by interposing the proximal side decreased diameter portion 46 between the pair of stent locking portions 34 and 35 of the shaft 20. In this state, the stent 12 is received inside the device 14 (sheath 16) (refer to Fig. 2). In this received state, the second region 72 to which the drug M is not applied is brought into contact with and supported by the protrusion portion 60. In this manner, the first region 70 to which the drug M is applied is brought into a state where the first region 70 is separated from the inner wall 26 of the sheath 16 (refer to Figs. 5A to 5C).

In a state where the stent 12 is received as described above, when the distal portion of the device 14 is delivered to stenosis inside the blood vessel and the sheath 16 is moved rearward relative to the shaft 20, the stent 12 is exposed from the distal opening 24a of the sheath 16. The exposure causes the stent 12 left in a contraction state to automatically expand radially outward. When the sheath 16 moves rearward, the protrusion portion 60 moves along the second region 72, thereby maintaining a state where the first region 70 is not brought into contact with the inner wall 26 of the sheath 16. Therefore, in a state of avoiding the detachment of the drug M which is caused by the interference of the inner wall 26, the stent 12 can be deployed inside the blood vessel.

That is, the system 10 and the stent 12 according to the present embodiment can considerably minimize the detachment of the drug M in such a way that the first region 70 to which the drug M is applied and the second region 72 to which the drug M is not applied are set to be located therein, and that only the second region 72 is brought into contact with and supported by the protrusion portion 60 of the sheath 16 (in other words, the first region 70 is not brought into contact with the inner wall 26 of the sheath 16).

The stent 12 deployed into the stenosis comes into contact with the inner surface of the blood vessel, and is caused to be indwelled in a state of spreading out the stenosis. Inside the stenosis where the stent 12 is indwelled, the stent 12 can stably elute the drug M through the first region 70 which maintains the quality of the drug M and effectively prevent restenosis of the treated portion.

Here, according the conventional living body lumen treatment system, the external force from the blood vessel or the like is applied to the device or the stent. Consequently, an approximately half range in the circumferential direction of the stent actively interferes with the inner side of the sheath, thereby causing a disadvantage in that the drug is peeled off from a section thereof. In contrast, in the system 10 and the stent 12 according to the present embodiment, the second regions 72 are set to be located at equal intervals in the circumferential direction of the stent 12. Accordingly, a section to which the drug M is not applied is equally dispersed. Therefore, it is possible to satisfactorily cover the second region 72 by causing the drug M to be eluted from the adjacent first region 70.

In addition, the stent 12 can be smoothly deployed into the blood vessel by setting the second region 72 to be located in the V-shaped lateral apex 40a where the distortion amount is large, and it is possible to more reliably prevent the detachment of the drug M.

Without being limited to the above-described embodiment, the system 10 and the stent 12 according to the present embodiment can adopt various configurations, as a matter of course. Hereinafter, some modification examples of the present invention will be described. Note that, in the following description, the same reference numerals are given to the same configuration elements or configurations having the same function as in the system 10 and the stent 12 according to the present embodiment, and thus detailed description thereof will be omitted.

### [First Modification Example]

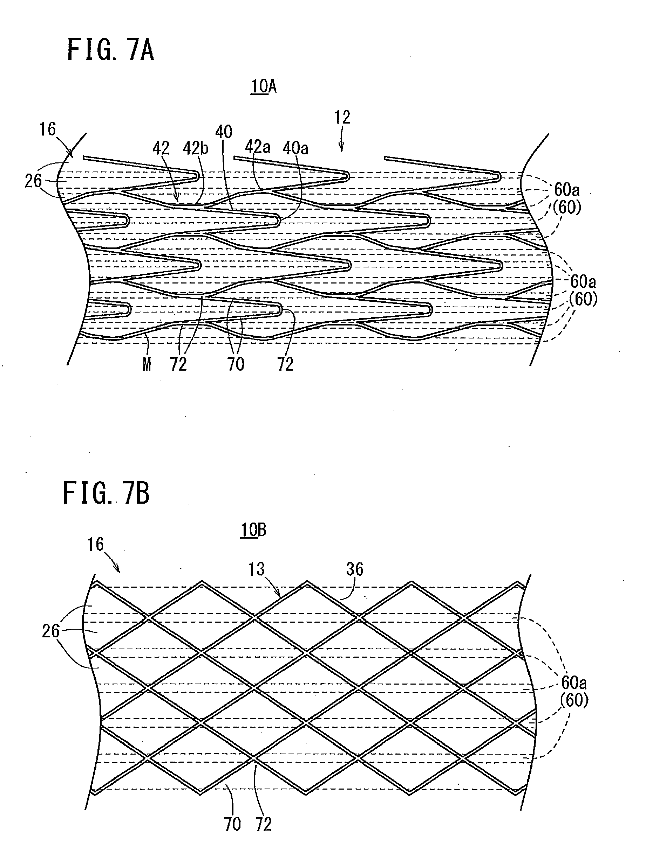

As illustrated in Fig. 7A, a system 10A according to a first modification example is different from the system 10 according to the present embodiment in that the second region 72 set in the stent 12 is set to be located in a row where the V-shaped lateral apex 40a and the wavy lateral apexes 42a and 42b are present, and that the multiple protrusion portions 60 disposed in the sheath 16 are disposed corresponding to the second region 72 of the stent 12.

Here, in the stent 12 configured to include the V-shaped framework portion 40 and the wavy framework portion 42, stress is likely to be applied to (have a large distortion amount) the wavy lateral apexes 42a and 42b subsequent to the V-shaped lateral apex 40a. Therefore, the second region 72 is also set to be located in the wavy lateral apexes 42a and 42b, and is configured to be supported by the protrusion portion 60. In this manner, it is possible to cause the sheath 16 to relatively and smoothly move relative to the stent 12 by more stably supporting the stent 12. Note that, even when the range of the second region 72 is widened, it is possible to satisfactorily elute the drug M after the stent 12 is indwelled by increasing an amount of the drug M applied to the first region 70.

### [Second Modification Example]

As illustrated in Fig. 7B, a system 10B according to a second modification example is different from the systems 10 and 10A according to the present embodiment and the first modification example in that a stent 13 is formed in a mesh shape. In this case, preferably, the protrusion portion 60 disposed in the sheath 16 supports intersection points of the wire 36 intersecting in a mesh shape. The second region 72 of the stent 12 may be set to be located in the axial direction along the intersection points, and the first region 70 may be set to be located in the wire 36 between the intersection points. In short, a shape of the stent received by the device is not particularly limited, and various shapes can be applied to the stent. The protrusion portion for supporting the stent can also be installed at a suitable position corresponding to the shape of the stent.

Hitherto, the present invention has been described with reference to the preferred embodiments. However, without being limited to the above-described embodiment, the present invention can be improved in various ways within the scope not departing from the gist of the present invention, as a matter of course. For example, without being limited to the configuration where the stent is indwelled in the lesion, the living body lumen treatment system may be applied to another structure body in addition to the stent. For example, the present invention can be applied to an atherectomy device having a structure body which is received in the sheath (tubular body) and is delivered so as to remove an atheroma inside the blood vessel.

## Claims

1. A living body lumen treatment system (10, 10A, 10B) comprising:
a tubular body (16) that can be delivered into a living body lumen, and that internally has a lumen (24) extending in an axial direction; and
a structure body (12, 12A, 12B, 13) that is received in a contraction state by the lumen (24), and that expands by being exposed from the tubular body (16) so as to come into contact with an inner surface of the living body lumen, wherein
a first region (70) to which a drug is applied and a second region (72) to which an application agent having an application form different from the drug is applied or to which the drug is not applied are set to be located on an outer surface of the structure body (12, 12A, 12B, 13),
the tubular body (16) has a protrusion portion (60) protruding inward beyond an inner wall (26) which configures the lumen (24), and
the structure body (12, 12A, 12B, 13) is received by the lumen (24) in such a way that the second region (72) is brought into contact with and supported by the protrusion portion (60) so as not to bring the first region (70) into contact with the inner wall (26).

2. The living body lumen treatment system (10, 10A, 10B) according to Claim 1, wherein
the protrusion portion (60) has a total length longer than a length in an axial direction of the structure body (12, 12A, 12B, 13), and is disposed along the axial direction of the tubular body (16), and
the first region (70) and the second region (72) extend along the axial direction of the structure body (12, 12A, 12B, 13), and are set at positions deviated from each other in a circumferential direction of the structure body (12, 12A, 12B, 13).

3. The living body lumen treatment system (10, 10A, 10B) according to Claim 1, wherein
a surface (60a) of the protrusion portion (60) that comes into contact with the second region (72) has a friction force lower than the inner wall (26).

4. The living body lumen treatment system (10, 10A, 10B) according to Claim 1, wherein
when the application agent is applied to the second region (72), the application agent satisfies at least one of physical properties selected from a lower friction force, higher elasticity, or higher rigidity, as compared to the drug.

5. The living body lumen treatment system (10, 10A, 10B) according to any one of Claims 1 to 4, wherein
the structure body (12, 12A, 12B, 13) is a stent which is configured to include an elastically deformable wire (36), and accordingly the stent is pressed and brought into a contraction state by the tubular body (16) when the stent is received inside the tubular body (16), and
the second region (72) is set to be located at a place where a distortion amount of the wire (36) is large in the contraction state.

6. A stent (12, 12A, 12B, 13) that is received in a contraction state by a lumen (24) of a tubular body (16) which can be delivered into a living body lumen, and that expands by being exposed from the tubular body (16) so as to be indwelled in the living body lumen, wherein
a first region (70) to which a drug is applied and a second region (72) to which an application agent having an application form different from the drug is applied or to which the drug is not applied are set to be located on an outer surface of the stent (12, 12A, 12B, 13), and
the stent (12, 12A, 12B, 13) is received by the lumen (24) in such a way that the second region (72) is brought into contact with and supported by a protrusion portion (60) protruding inward beyond an inner wall (26) configuring the lumen (24) so as not to bring the first region (70) into contact with the inner wall (26).

7. The stent (12, 12A, 12B, 13) according to Claim 6, wherein
the first region (70) and the second region (72) extend along an axial direction of the stent (12, 12A, 12B, 13), and are set at positions deviated from each other in a circumferential direction of the stent (12, 12A, 12B, 13).

8. The stent (12, 12A, 12B, 13) according to Claim 6, wherein
when the application agent is applied to the second region (72), the application agent satisfies at least one of physical properties selected from a lower friction force, higher elasticity, or higher rigidity, as compared to the drug.

9. The stent (12, 12A, 12B, 13) according to any one of Claims 6 to 8, wherein
the stent (12, 12A, 12B, 13) is configured to include an elastically deformable wire (36), and accordingly the stent is pressed and brought into a contraction state by the tubular body (16) when the stent is received inside the tubular body (16), and
the second region (72) is set to be located at a place where a distortion amount of the wire (36) is large in the contraction state.
